# EUROPEAN PATENT APPLICATION

(11) **EP 1 350 529 A1**
(43) Date of publication of application: **08.10.2003**
(21) Application number: 03005098.3
(22) Date of filing: 07.03.2003
(51) Int. Cl.: A61M 5/32

(54) **Medical device**

(30) Priority: 04.04.2002 US 369607 P
(71) Applicant: Becton, Dickinson and Company, Franklin Lakes, New Jersey 07417-1880 (US)
(72) Inventor: Hwang, Charles G., Ridgewood, New Jersey 07450 (US); Swenson, Kirk D., North Caldwell, New Jersey 07006 (US)
(74) Representative: von Kreisler, Alek, Dipl.-Chem.

(57) **Abstract**

A safety shield (10) assembly for an intravenous apparatus is provided including a needle (12), a needle hub (14), a forward shield (26), a guide element (24), and a locking member (56). The needle hub (14) is in fluid communication with a proximal end of the needle and includes a pair of flexible wings (30). The shield (26) includes a distal blunting end (18) having a distal aperture (20) and a proximal needle passageway (22). The shield (26) is moveable from a retracted position, in which the needle tip is exposed through the distal aperture (20), to an extended position, in which the needle tip is withdrawn from the aperture and is covered by the blunting end (18). The guide element (24) is arranged on the hub (14) for slidably accommodating the shield (25) for movement from the retracted position to the extended position. The locking member (56) locks the forward shield with respect to the guide element (24) in the extended position, upon slidable movement of the shield (26) into the extended position.

## Description

### FIELD OF THE INVENTION

The present invention relates generally to needle shield assemblies for blood collection/intravenous infusion devices. More particularly, the present invention relates to a needle shield assembly having a needle shield that may be activated by a single-handed operation in order to avoid accidental needle sticks.

### DESCRIPTION OF THE PRIOR ART

A conventional IV infusion or blood collection assembly includes an elongated small gauge plastic flexible tubing material having a disposable needle and a body or hub for holding the needle on one end. Usually, the hub is adhered to one end of the flexible tube by a friction fit. The hub includes wings extending on either side for the phlebotomist or user to grasp and position the hub for inserting the needle into a patient. Such assemblies may be used for infusing medication into a patient or for collecting blood from a patient. Generally, at the end of the flexible tube opposite the needle, is a female connection for connecting supplies of fluid to be infused or for connecting apparatus for collecting blood, as required.

After the needle of the assembly has been withdrawn from a patient, protection of the used needle tip becomes important. Accidental sticks with a used needle can transmit bloodborne disease such as hepatitis, AIDS and other similar diseases. As a result, there is a present need for methods and devices to enclose the used disposable needle by providing some sort of covering for the used needle tip after it has been withdrawn from the patient.

Many needle guards are known including placing a separate needle cap over the needle after use, positioning a sliding shield distally over the used needle, or hiding the withdrawn needle within a hollow needle bearing holder. Many current needle guards employ a two handed technique. Such a two handed technique is awkward to use and may still pose a risk of accidental needle stick.

In addition to safety shielding devices for needles which require two-handed operation, the art has also seen the use of needle shield devices which are automatic and do not require one-handed or two-handed activation. However, many of these shields can be activated unintentionally, thus rendering the unused instrument unsuitable for its intended purpose.

There exists a need for a simple, straight-forward, reliable, easily fabricated needle assembly which is self-contained, capable of single-handed activation, and can be used with blood collection and intravenous delivery devices.

### SUMMARY OF THE INVENTION

The present invention provides a safety shield which can be activated using a one-handed technique for use with blood collection devices.

A safety shield assembly for an intravenous apparatus is provided including a needle, a needle hub, a forward shield, a guide element and a locking member. The needle hub is in fluid communication with a proximal end of the needle and includes a pair of flexible wings. The shield includes a distal blunting end having a distal aperture and a proximal needle passageway. The shield is moveable from a retracted position, in which the needle tip is exposed through the distal aperture, to an extended position, in which the needle tip is withdrawn from the aperture and is covered by the blunting end. The guide element is arranged on the hub for slidably accommodating the shield for movement from the retracted position to the extended position. The locking member locks the forward shield with respect to the guide element in the extended position, upon slidable movement of the shield into the extended position.

A safety shield assembly for an intravenous apparatus is provided including a needle, a needle hub, a forward shield, and a guide element. The needle hub is in fluid communication with a proximal end of the needle and includes a pair of flexible wings. The forward shield includes a distal blunting end, a proximal finger contact surface, an arched barrier arm interposed between the blunting end and the finger contact surface, and a wedge-shaped locking projection having a wider proximal end than a distal end. The shield is moveable from a retracted position, in which the needle tip is exposed through the distal aperture, to an extended position, in which the needle tip is withdrawn from the aperture and is covered by the blunting end. The guide element is arranged on the hub for slidably accommodating the forward shield for movement from the retracted position to the extended position. The guide element includes a guide groove having a pair of inwardly directed projections, whereby the inwardly directed projections engage the locking projection to prevent retrograde movement when the forward shield is in the extended position.

### BRIEF DESCRIPTION OF THE DRAWINGS

FIG. 1 is a perspective top view of a conventional blood infusion set, without a needle shield assembly;

FIG. 2 is a top view of the assembly of FIG. 1;

FIG. 3 is a side view of a safety shield assembly according to the present invention with the shield in a retracted position and attached to a blood collection or intravenous tube;

FIG. 4 is a side view of the needle assembly of FIG. 3 with the shield in an extended position;

FIG. 5 is a side view of a safety shield assembly according to the present invention showing the shield in an extended position with the needle tip out of alignment with the aperture of the blunting end of the forward shield;

FIG. 6 is a top plan view of a barrier arm according to the present invention;

FIG. 7 is a side view of a safety shield assembly according to the invention including a retaining projection and color segments;

FIG. 8 is a side view of a safety shield assembly according to the present invention with the shield in the retracted position and including a spring member and releasable latch for assisting forward movement of the forward blunting member;

FIG. 9 is a side view of a safety shield assembly according to the present invention with the shield in the extended position and having a spring member and releasable latch for assisting forward movement of the forward blunting member;

FIG. 10 is a front view of a cross section of a guide element according to an embodiment of the invention, along lines X-X of FIG. 4.

FIG. 11 is a perspective plan view of a needle cap according to the invention; and

FIG. 12 is a cross-sectional view of the needle cap of FIG. 11 along lines XI-XI of FIG. 11.

### DETAILED DESCRIPTION OF THE PREFERRED EMBODIMENTS

While this invention is satisfied by embodiments in many different forms, there is shown in the drawings and will herein be described in detail preferred embodiments of the invention with the understanding that the present disclosure is to be considered as exemplary of the principles of the invention and is not intended to limit the invention to the embodiments illustrated. The scope of the invention will be measured by the appended claims and their equivalents.

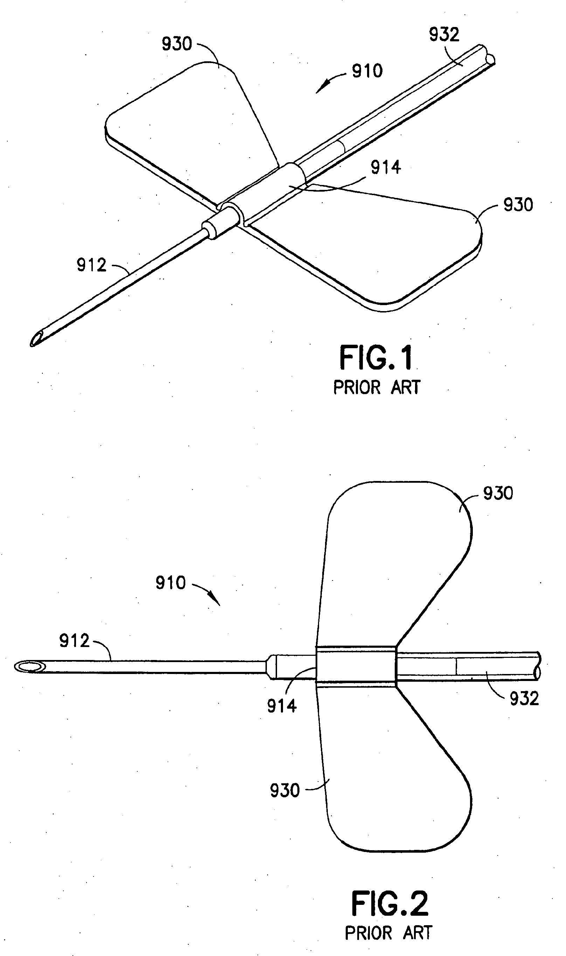

Referring to the drawings in which like characters refer to like parts throughout the several views, FIGS. 1 and 2 show a conventional IV infusion set **910**, including a needle **912** embedded in a needle hub **914** toward the proximal end of the needle **912**. Flexible tubing **932** extends from the proximal end of the hub **914** which is conventional tubing for allowing the user to connect to supplies of infusion liquids or collection tubes for blood collection. The IV infusion set generally includes flexible wings **930** attached to and projected outwardly from the hub **914.** The flexible wings **930** are usually integral with the hub **914.**

The safety shield assembly of the present invention includes a safety shield in cooperative engagement with a guide member. The shield slides over the needle to protect the needle tip. Features of the invention include one or more retaining members to keep the shield in place in a retracted position and to keep the needle tip available for use. A locking member is provided to keep the shield in place over the needle tip in an extended position after the needle has become contaminated. These and other features of the invention are described in detail below.

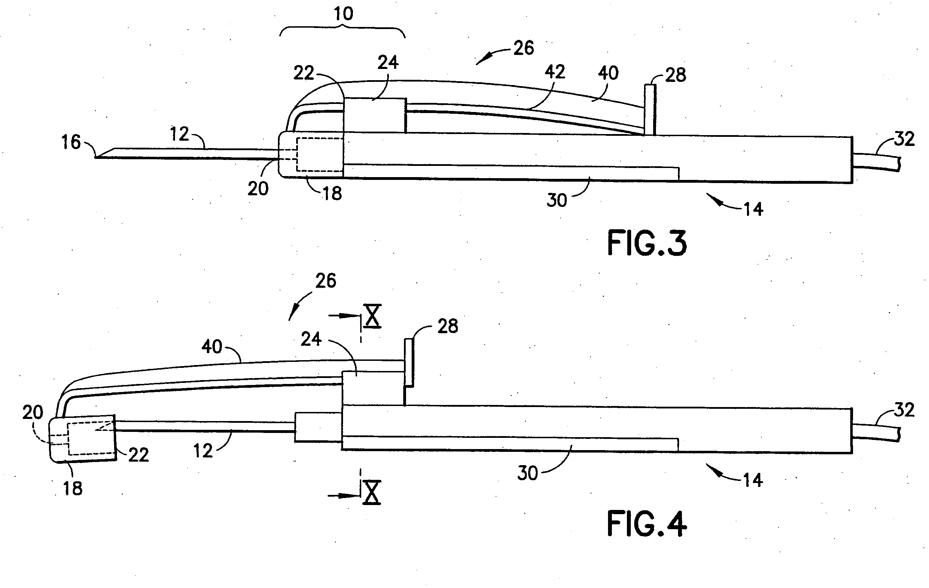

Referring now to FIGS. 3 and 4, a safety shield assembly **10** according to the present invention, is shown. The safety needle assembly includes a needle **12** having a proximal end, a distal end, with a lumen therethrough, and a sharp distal tip **16**. A needle hub **14** is arranged at the proximal end of the needle **1**2 and has an interior cavity in fluid communication with the lumen. The needle hub **14** has a distal end, a proximal end and a main body therebetween. A pair of flexible wings **30** are positioned on the main body of the hub **14** for guiding the needle **12** during venipuncture.

In this configuration, a guide element **24** is a separate part connected to the needle hub **14**. In this case, the guide element **24** is connected toward a distal end of the hub **14.** The guide element **24** includes a guide groove (not shown) and is generally axially oriented about the needle and dimensioned to accept a forward shield **26**. The guide element **24** may be attached to the hub **14** through the use of any suitable joining technique such as adhesives, ultrasonic welding and the like. For low volume production, attaching the guide element to an existing hub **14**, is desirable from a cost standpoint. It is within the purview of the present invention to include an integrally molded one-piece hub **14** and guide element. For high volume production it may be desirable that the needle hub **14** and guide element **24** be of one-piece integrally molded thermoplastic.

Referring again to FIGS. 3 and 4, the forward shield **26** includes an elongate barrier arm **40** having a blunting end **18** for protecting the needle tip **16** arranged toward the distal end of the arm **40**. The blunting end **18** has a distal aperture **20** and a proximal needle passageway **22** for accommodating the needle **12**. The barrier arm **40 i**s positioned within the guide groove **34** (not shown). The needle **12** is positioned within the proximal needle passageway **22** of the blunting end **18**.

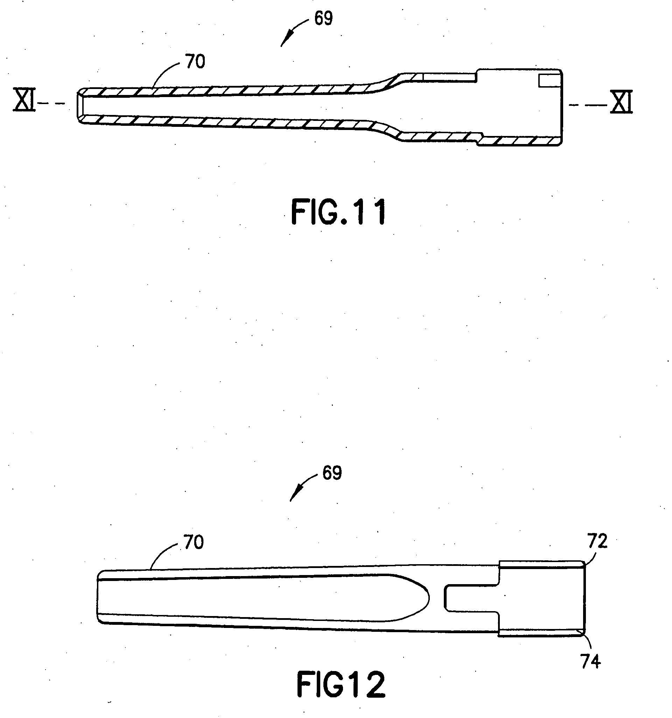

As can be seen in FIG. 10, which is a partial cross-sectional view of lines X-X of FIG. 4, the guide groove **34** is sized to accommodate a portion of the barrier arm **40** of the shield **26**. The barrier arm **40** of the forward shield **26** is thus slidably connected to the guide element **24** via the guide groove **34**. The barrier arm **40** includes a rib **42** which fits into the guide groove **34.** The forward shield **26** is movable from at least a retracted position as illustrated in FIG. 3, wherein the needle tip **16** passes completely through the blunting end **18** and is exposed through the aperture **20**, to an extended position, in which the needle tip **16** is withdrawn from the aperture **20** and resides inside the blunting end **18**, as illustrated in FIG. 4.

Preferably, the barrier arm **40** and blunting end **18** of the forward shield **26** are integrally molded of the same material. However, the barrier arm **40** and the blunting end **18** can be separately formed and joined together by any suitable means such as adhesive, ultrasonic welding and frictional or snap-fit type engagement. The barrier arm **40** and blunting end **18** can also be separately formed and connected by a separate element such as a metal clip (not shown).

A proximal end of the forward shield **26** includes a finger contact surface **28** to allow the single-handed movement of the barrier arm **40** from the retracted position of FIG. 3 to the extended position of FIG. 4. In this preferred embodiment, finger contact surface **28** is provided on the proximal end of the elongate barrier arm **40**. Application of a digital pressure to the finger contact surface **28** moves the forward shield **26** into the extended position. The forward shield **26** slides along the guide groove **34** after the needle **12** has been used. Thus, a one handed method can be used to place the forward shield **26** in the extended position and shield the needle tip **16** so as to prevent injury. This position for the finger contact surface is preferred because it is the farthest position from the sharp needle tip **16**.

The barrier arm **40** can be single-handedly advanced from the retracted position to the extended position by holding the hub **14** in one hand and pushing on finger contact surface **28** with the thumb or the index finger of the holding hand. Dorsal placement of the guide element **24** and the forward shield **26** with respect to the needle **12** and hub **14** allows for the wings **30** to be used unimpeded during placement of the needle in the patient. The ability to allow single-handed operation is significant since it allows the person administering the injection to use the other hand for other purposes such as applying pressure to a vein to prevent bleeding.

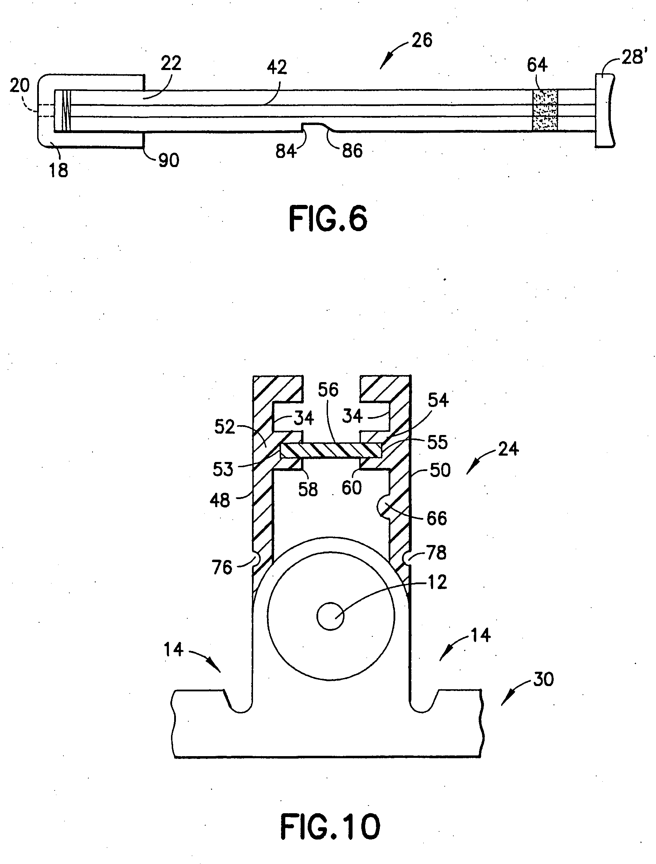

Referring now to FIGS. 6 and 10, showing a top view of the guide element **24** and a front cross-sectional view of the forward shield **26,** respectively, a further aspect of the invention is shown. In this aspect, a further retaining member is provided for retaining the shield **24** in the retracted position. The further retaining member includes a recess **84** in the barrier arm **40** for accepting a retaining projection **66** on the guide element **24** when the barrier arm **40** is in the retracted position. In this position, the barrier arm **40** does not have to be tightly contained within the guide groove **34**. Accordingly, the plastic components are not stressed during the long period of time between manufacture and use.

Upon applying force to the barrier arm **40** to move it distally, retaining projection **66** rides up a ramped portion **86** of retaining recess **84** to immediately increase the force necessary to move the barrier arm **40** in a distal direction. This design helps to retain the barrier arm **40** in the retracted position. The shapes of retaining projection **66** and the ramped portion **86** may be configured so that there is an audible click when retaining projection **66** leaves retaining recess **84**, further communicating to the healthcare worker that the barrier arm **40** is in motion toward the second extended position.

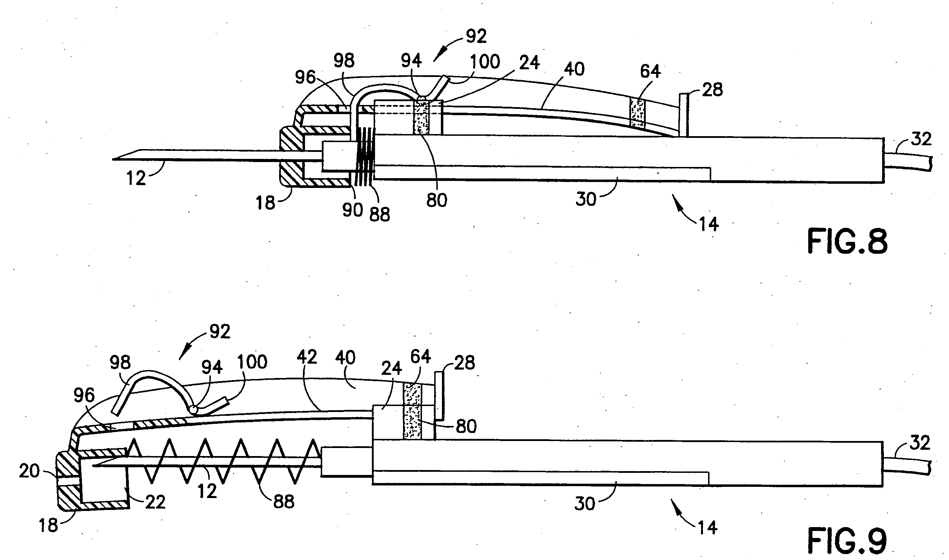

Referring now to FIG. 7, a first retaining member for releasably retaining the barrier arm **40** in the first retracted position is shown. This function can be accomplished by dimensioning the barrier arm **40** and the groove **34** in the guide element **24** to have a frictional fit. In this preferred aspect, the barrier arm **40** is arched. Specifically, a distal end and a proximal end of the barrier arm **40** define longitudinal axes A1 and A2, respectively. A1 and A2 are at an obtuse angle "α" with respect to each other. The retaining arm **34** defines a substantially rectilinear opening for accommodating a straight member. The non-straight or arched configuration of the barrier arm **40**, when it enters a groove **34** sized for a straight barrier arm, provides a frictional relationship which keeps the barrier arm **40** from moving distally under less than the desired force.

Additionally, the present invention includes a locking member for preventing retrograde movement of the barrier arm **40** from the extended position. The locking member is activated by movement of the barrier arm **40** into the extended position.

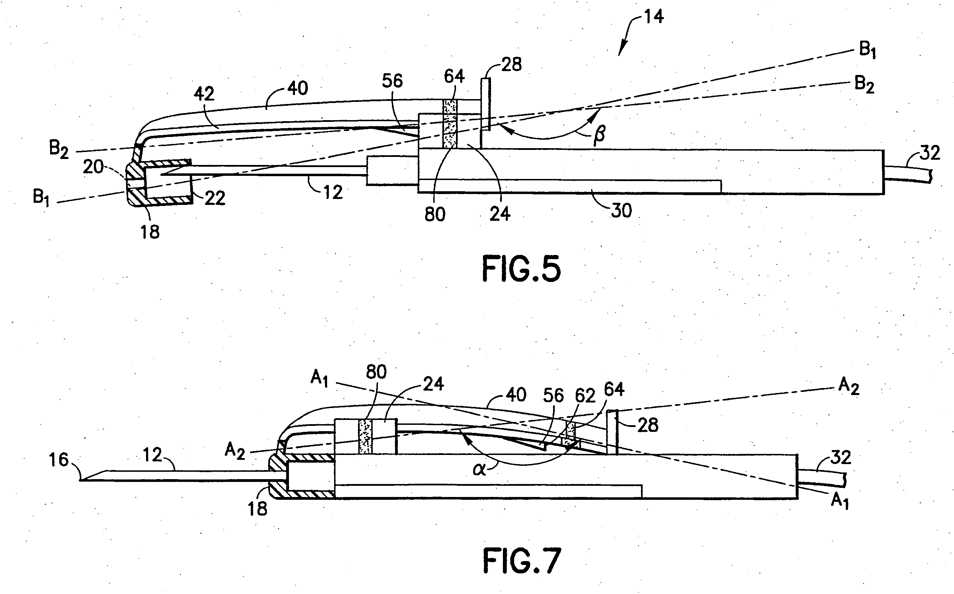

Referring now to FIG. 5, in one aspect of the invention, a locking member is shown. The locking member locks the barrier arm **40** in the extended position. The locking member is essentially the same as the aforementioned first retaining member. The locking member includes the distal end of the barrier arm **40** defining a distal longitudinal axis B1 and the proximal end of the barrier arm defining a proximal longitudinal axis B2. The barrier arm **40** is arched so that the distal longitudinal axis and the proximal longitudinal axis are at obtuse angle "β" with respect to each other, when the barrier arm is in the second extended position. Due to the arched configuration of the barrier arm **40**, the distal end of the needle is out of alignment or "off center" with the distal aperture **20** when the forward shield **26** is in the extended position. An attempt to move the barrier arm **40** from the second extended position will cause the sharpened distal tip **16** of the needle to embed itself into an interior wall of the blunting end **18** of the forward shield **26** to help prevent re-exposing the needle tip **16**. This is an important feature of the present invention because the locking member is essentially part of the forward shield **26**. As a result, additional parts and structures such as latches, ledges, triggers and the like, which add to cost and complexity and can adversely affect reliability, are avoided.

Referring now to FIGS. 5 and 10, a further locking member of the invention is shown. In this embodiment, the needle shield assembly includes additional structure features to further resist retrograde motion of the barrier arm **40** from the extended position to the retracted position. In this embodiment, the guide groove **34** and the barrier arm **40** include a combination of projections and recesses which allow the barrier arm **40** to slide along the guide groove **34**, in a distal direction, by application of digital pressure on finger contact surface **28**. However, the projections and recesses are configured such that returning the barrier arm **40** to the retracted position is not possible using forces normally associated with operating the invention.

In FIG. 10, a partial front cross-sectional view of line X-X of FIG. 4 is shown. The guide element **24** includes sidewalls **48** and **50** having generally opposed inwardly directed projections **52** and **54,** respectively. The projections **52** and **54,** include indents **53** and **55,** configured to interact with a raised locking projection **56** on barrier arm **40** as shown in FIG. 5. The raised locking projection **56** is wedge-shaped and is wider at its proximal end than at its distal end.

When force is applied to the barrier arm **40** to move the guide element **24** from the first retracted position to the second extended position, then distal motion of the barrier arm **40** will cause raised locking projection **56** to contact indents **53** and **55** on projections **52** and **54** of the guide element **24**. Additional force will be required to force the raised locking projection **56** through the indents in the projections **52** and **54**. This action expands the space between the sidewalls **48** and **50** of the shield **26** to accommodate the locking projections **56**. Once this occurs, the projections **52** and **54** will return to their original position and an edge **58** on projection **52**, and an edge **60** on projection **54** will form a locking relationship with respect to back wall **62** of raised locking projection **56** to prevent retrograde movement. This aspect of the invention can optionally be used with the previously described locking member to help prevent retrograde movement of the barrier arm **40** from the second extended position. There is no particular limitation on placement of the recesses and projections. For example, the recesses and projections may be placed on the top of the guide element and barrier arm, respectively.

In a further aspect of the invention, the projections **52** and **54** on the guide element and raised locking projection **56** on the barrier arm **40** are configured so that when the barrier arm **40** is moved distally into the second extended position, the projections **52** and **54** will snap past the end of raised locking projection **56**, making an audible sound to provide an audible indication that the barrier arm **40** is moved into the second extended position. It is preferred that two projections be provided on the guide element, however, only one projection will work and is within the purview of the present invention.

In a further aspect of the invention, the assembly is provided with color segments to visually verify when the barrier arm **40** is in the second extended position. Referring now to FIGS. 5 and 7, a color segment **64** on barrier arm **40** and a color segment **80** on the guide element **24** are shown. When the barrier arm **40** is in the retracted position, as shown in FIG. 7, then the color segment **64** on barrier arm **40** is in non-alignment with the color segment **80** on the guide element **24**. When the barrier arm **40** is in the extended position, the color segment **64** is in alignment with the color segment **80** of the guide element **24**. Alignment of the color segments **64** and **80** forms a continuous transverse color bond. This indicates the assembly **10** is fully in the extended position, and therefore safer for disposal. In one embodiment, the guide element **24** includes two generally opposed color segments on the guide element **24**. Any color, including black and white, can be used to form the color segments. However, green is preferred, because it is generally accepted to indicate safety.

The needle shield assembly of the invention provides an easy to use shield which can be operated manually using a one handed technique. The user is protected from inadvertent needle sticks by pushing the shield distally until the blunting end covers the needle tip. However, it is also useful to have the shield move automatically over the needle tip upon release of the shield from the hub. To this end, a further aspect of the invention is provided including a spring activated assist mechanism for distally moving the barrier arm **40** into the second extended position.

Referring now to FIGS. 8 and 9, a spring **88** is arranged between the guide element **24** and a proximal edge **90** of the blunting end **18.** The spring **88** wraps around the needle **12** and is held in place by a pivoting latch **92**. The pivoting latch **92** includes a pivot arm **98,** a pivot lever **100** and a pivot point **94** therebetween. The pivoting latch **92** is anchored onto the barrier arm **40** at pivot point **94.** In a closed position, pivot arm **98** is arranged in an opening or channel **96** on the barrier arm **40** so as to hold the spring **88** in a biased state.

The pivoting latch **92** is activated by putting downward pressure on the pivot lever **100** so as to elevate the pivot arm **98** from the opening **96** and release the spring **88**. Once released, the spring **88** exerts a compression force against the proximal end **90** of the blunting end **18**. The compression force thereby assists in moving the forward shield **26** into the second extended position. In this embodiment, the user does not have to manually push the shield **24** distally. Rather, activation of the pivot lever is all that is required to safely guard the needle.

Referring now to FIGS. 10, 11 and 12, a needle cap is shown. A further aspect of the present invention includes a needle cap **69** having a side wall **70** which defines a cavity therebetween for receiving the needle **12**. A proximal end of the needle cap **70** includes an opening configured to releasably engage a portion of the guide element. The cap serves to hold the needle shield in a needle protecting position while the barrier arm **40** is in the first retracted position. The needle cap **70** is configured so that when the barrier arm **40** is in the second extended position, the needle cap **70** cannot be connected to the needle assembly.

Needle cap **70** is removably connected to the guide element **24** through action of projections **72** and **74** at the proximal end of the needle cap **70** which interact with complimentary recesses **76** and **78** on the guide element **24**. The needle cap **70** is configured to accept the barrier arm **40** so that there can be a substantially complete shielding of the needle. Accordingly, the needle cap **70** can effectively protect the needle from damage and inadvertent contact from the time of manufacture until the time of use, at which point the cap **70** is appropriately discarded. After the forward shield **26** is moved to the second extended position, the needle cap **70** can no longer be used with the safety shield assembly **10** of the present invention.

An alternate aspect of the invention includes a barrier arm **40** having a proximal end and a distal end. The barrier arm **40** includes a blunting end **18** having a distal end, a proximal end, and a needle passageway **22** therethrough. This alternate needle assembly functions in substantially the same manner as the needle assembly taught in FIGS. 3-11 except that the needle passageway **22** is generally continuous throughout the length of the forward shield. When the forward shield **26** is moved to the second extended position, the sharpened tip **16** of the needle **12** will be pressing firmly against the sidewalls of the needle passageway **22** so that any attempt to move the barrier arm **40** from the second extended position to the first retracted position will cause the needle to embed itself in the sidewall of needle passageway **22** to inhibit proximal movement of the barrier arm.

Another alternative aspect of the safety shield assembly includes the hub **14** and the guide element **24** being integrally formed of one-piece construction, preferably of thermoplastic material, most preferable a clear transparent thermoplastic material.

The needle assembly of the present invention is suitable for use with a wide variety of medical devices, including blood collection devices. The invention is illustrated in FIGS. 3-8 as being used with blood collection and/or fluid delivery tubing **32**.

Various other modifications to the foregoing disclosed embodiments will now be evident to those skilled in the art. Thus, the particularly described preferred embodiments are intended to be illustrative and not limited thereto. The true scope of the invention is set forth in the following claims.

## Claims

1. A safety shield assembly for an intravenous apparatus, comprising:
a needle having a distal needle tip;
a needle hub in fluid communication with a proximal end of said needle and including a pair of flexible wings;
a forward shield including a distal blunting end having a distal aperture and a proximal needle passageway, wherein said shield is moveable from a retracted position in which said needle tip is exposed through the distal aperture to an extended position in which said needle tip is withdrawn from said aperture and is covered by said blunting end;
a guide element arranged on said hub for slidably accommodating said forward shield for movement from said retracted position to said extended position; and
locking means for locking said forward shield with respect to said guide element in said extended position, upon said slidable movement of said forward shield into said extended position.

2. The assembly of claim 1, wherein said forward shield further comprises a finger contact surface.

3. The assembly of claim 2, wherein said forward shield further comprises a barrier arm interposed between said distal blunting end and said finger contact surface.

4. The assembly of claim 3, wherein said guide element further comprises a guide groove in slidable connection with the barrier arm.

5. The assembly of claim 4, wherein said locking means comprises at least one inwardly directed projection on said guide groove, said projection and said shield being configured so as to allow distal movement of said shield along said guide groove and to prevent retrograde movement of said shield from said extended position.

6. The assembly of claim 5, wherein said shield further comprises a wedge-shaped locking projection having a wider proximal end than a distal end, said locking projection being capable of exerting a lateral expanding force on said projection of said guide groove when said shield is moved distally.

7. The assembly of claim 6, wherein said locking projection is on said barrier arm.

8. The assembly of claim 7, wherein said distal movement of said locking projection of said barrier arm beyond said projection of said guide groove emits an audible sound.

9. The assembly of claim 6, wherein said locking means comprises two substantially opposed inwardly directed projections.

10. The assembly of claim 9, wherein each of said two projections includes an indent for accommodating said locking projection of said shield.

11. The assembly of claim 4, wherein said barrier arm is arched, said guide groove defines a substantially rectilinear opening, and whereby movement of said shield along said guide groove into said extended position moves said needle tip off-center with respect to said distal aperture.

12. The assembly of claim 1, further comprising visual detection means for determining when said shield is in said extended position.

13. The assembly of claim 12, wherein said visual detection means includes a first color segment on said shield and a second color segment on said guide element, whereby said first and second color segments form a transverse color band when said shield is in said extended position.

14. The assembly of claim 1, further comprising retaining means for releasably retaining said shield in said retracted position.

15. The assembly of claim 14, wherein said retaining means comprises a retaining projection on said guide element and a corresponding ramped recess on said shield, whereby said retaining projection and recess are in releasable engagement when said shield is in said retracted position.

16. The assembly of claim 1, further comprising a removable needle cap having a polygonal sidewall defining a cavity for receiving said needle, wherein a proximal end of said cap releasably engages a portion of said guide element when said shield is in said retracted position.

17. The assembly of claim 16, wherein said polygonal sidewall is configured so as to be incapable of engaging said guide element when said shield is in said extended position.

18. The assembly of claim 1, wherein said blunting end includes an access port, the assembly further comprising:
a spring arranged concentrically about said needle and interposed between a distal end of said needle hub and said blunting end, and
a latch including a distal arm, a proximal lever, and a pivot point therebetween, said latch being pivotally connected to said shield at said pivot point, wherein said latch is moveable between a closed position, in which said pivot arm extends through said access port to retain said spring and an opened position in which said pivot arm releases said spring onto said blunting end.

19. A safety shield assembly for an intravenous apparatus, comprising:
a needle;
a needle hub in fluid communication with a proximal end of said needle and including a pair of flexible wings;
a forward shield including a distal blunting end, a proximal finger contact surface, an arched barrier arm interposed between said blunting end and said finger contact surface, and a wedge-shaped locking projection having a wider proximal end than a distal end, wherein said shield is moveable from a retracted position in which said needle tip is exposed through said distal aperture to an extended position in which said needle tip is withdrawn from said aperture and is covered by said blunting end; and
a guide element arranged on said hub for slidably accommodating said shield for movement from said retracted position to said extended position, said guide element including a guide groove having a pair of inwardly directed projections, whereby said inwardly directed projections engage said locking projection to prevent retrograde movement when said shield is in said extended position.

20. The assembly of claim 19, wherein said blunting end includes an access port, said assembly further comprising:
a spring arranged concentrically about said needle and interposed between a distal end of said needle hub and said blunting end, and
a latch including a distal arm, a proximal lever, and a pivot point therebetween, said latch being pivotally connected to said shield at said pivot point, wherein said latch is moveable between a closed position in which said pivot arm extends through said access port to retain said spring and an opened position in which said pivot arm releases said spring onto said blunting end.
